(19) **Europäisches Patentamt**
**European Patent Office**
**Office européen des brevets**

(11) **EP 2 774 570 B1**

(12) **EUROPEAN PATENT SPECIFICATION**

(45) Date of publication and mention
of the grant of the patent:
**18.05.2016 Bulletin 2016/20**

(51) Int Cl.:
*A61B 42/00* (2016.01)    *A61L 31/08* (2006.01)
*B65D 83/08* (2006.01)    *B05B 11/00* (2006.01)
*B65D 81/28* (2006.01)

(21) Application number: **14158296.5**

(22) Date of filing: **07.03.2014**

(54) **Glove packaging having antimicrobial barrier**

Handschuhverpackung mit antimikrobieller Barriere

Emballage pour gants ayant une barrière antimicrobienne

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.03.2013 US 201313790091**

(43) Date of publication of application:
**10.09.2014 Bulletin 2014/37**

(60) Divisional application:
**15003537.6 / 3 011 982**

(73) Proprietor: **Medline Industries, Inc.
Mundelein, IL 60060 (US)**

(72) Inventors:
• **Yao, Min
Vernon Hills, Illinois 60061 (US)**
• **Amdur, Samuel T.H.
Libertyville, Illinois 60048 (US)**

(74) Representative: **Grünecker Patent- und Rechtsanwälte
PartG mbB
Leopoldstraße 4
80802 München (DE)**

(56) References cited:
**WO-A1-2007/004562    WO-A1-2009/129182
US-A1- 2004 084 378    US-A1- 2006 177 489
US-A1- 2006 204 452    US-A1- 2009 130 157**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

The present disclosure relates generally to packaging for gloves. More particularly, the present disclosure relates to packaging for gloves having a barrier for protecting the gloves from contamination from microorganisms and other undesirable materials or contaminants, and methods for making the packaging.

### BACKGROUND

Gloves are widely used as a protective measure and have become mandatory in many industries and nearly all medical and surgical settings. In particular, disposable gloves are required as a means for protecting medical and surgical staff from coming into contact with bodily fluids during surgical procedures, medical examinations, laboratory testing and other medical procedures. Disposable gloves have traditionally been made of rubber materials such as latex, thermoplastic materials such as vinyl, and other natural and synthetic materials.

Many gloves are provided in packaging having a cavity for holding the gloves. The packaging includes an opening for removing the gloves from the packaging. The opening is typically revealed by removing a perforated portion of the packaging to access the gloves. Once the perforated portion of the packaging is removed to reveal the opening, the gloves are exposed to the ambient environment. As the ambient environment may contain microorganisms, pathogens, small airborne particles of dust and debris and other air contaminants, the gloves contained in the packaging may be exposed to undesirable materials or contaminants that may contaminate the gloves while they are in the packaging.

Thus, there exists a need for a glove packaging that includes a barrier to protect gloves contained within the glove packaging from microorganisms, airborne particles and other materials or contaminants that may contaminate the gloves prior to removal from the packaging. Preferably, the barrier can also destroy any microorganisms, pathogens or other materials or contaminants that come in contact with the barrier to further reduce the possibility of contamination.

Document WO 2009/129182 A1 discloses a glove packaging having an antimicrobial barrier according to the preamble of independent claims 1 and 11.

### SUMMARY

According to one embodiment of the present concepts, a packaging for gloves comprises a container having a cavity for holding the gloves, as defined in appended claim 1. The container includes an opening for removing the gloves from the container. The packaging also comprises a barrier including an antimicrobial material positioned to cover at least a portion of the opening of the container.

In another embodiment of the present concepts, a method for making a packaging for gloves comprises providing a container having a cavity for holding the gloves, as defined in appended claim 11. The container includes an opening for removing the gloves from the container. The method further comprises providing at least a first barrier having an antimicrobial material and attaching the first barrier to the container such that the barrier at least partially covers a portion of the opening of the container.

In yet another embodiment of the present concepts, a container for holding a plurality of gloves comprises a body portion, an opening and an antimicrobial film covering the opening. The antimicrobial film includes an aperture to facilitate removal of the gloves from the opening of the container.

The above summary of the present concepts is not intended to represent each embodiment or every aspect of the present concepts. The detailed description and Figures will describe many of the embodiments and aspects of the present concepts.

### BRIEF DESCRIPTION OF THE DRAWINGS

The foregoing and other advantages of the invention will become apparent upon reading the following detailed description and upon reference to the drawings.

FIG. 1 is a perspective view of a glove packaging according to one embodiment of the present concepts.
FIG. 2 is a top view of the glove packaging illustrating an antimicrobial barrier.
FIG. 3 is a side view of a barrier including an antimicrobial material.
FIG. 4 is a side view of another embodiment of a barrier including an antimicrobial material.

While the invention is susceptible to various modifications and alternative forums, specific embodiments have been shown by way of example in the drawings and will be described in detail herein. It should be understood, however, that the invention is not intended to be limited to the particular forms disclosed. Rather, the invention is to cover all modifications, equivalents, and alternatives falling within the scope of the appended claims.

## DESCRIPTION OF ILLUSTRATIVE EMBODIMENTS

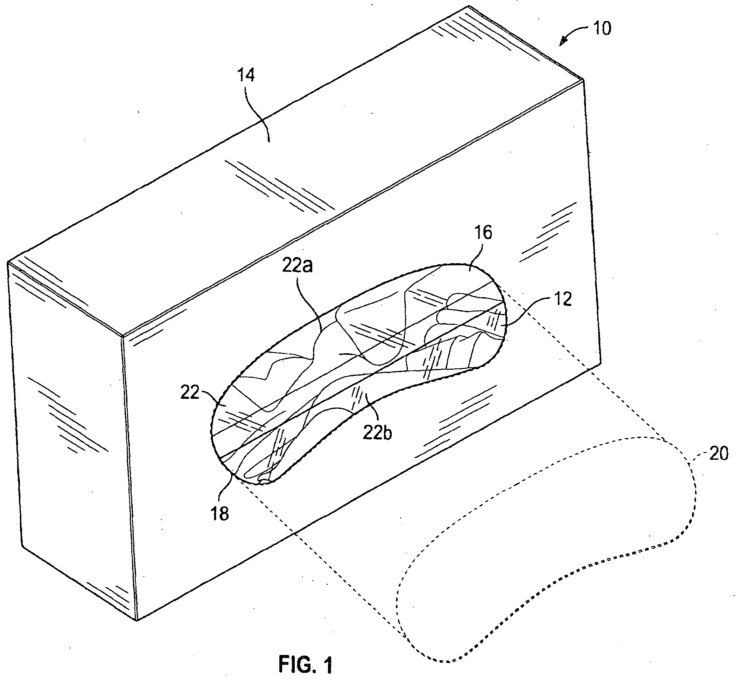

FIG. 1 illustrates a packaging 10 for gloves 12. The packaging 10 comprises a container 14 having a cavity 16 for holding the gloves 12. The container 14 includes an opening 18 for removing the gloves 12 from the container 14. The opening 18 may be in the form of different shapes, such as a circle, an oval, a square, a rectangle, or any variation of such shapes, such that a user may insert his or her hand through the opening 18 to remove one or more of the gloves 12. The opening 18 may initially be covered by a removable segment 20 that is initially formed as part of the container 14. The removable segment 20, which may be a perforated segment, is removable from the packaging 10 by a user once the packaging 10 is ready to be opened by tearing the removable segment 20 away from the packaging 10. The removable segment 20 is generally discarded after it is removed from the packaging 10. In addition to a perforated segment, the removable segment 20 may include an adhesive segment that is removable from the packaging 10.

The container 14 and removable segment 20 may be comprised of a variety of materials or combinations of materials, such as paper, plastic or fabric. The gloves 12 may include medical and/or surgical disposable gloves that are comprised of rubber materials such as latex, thermoplastic materials such as vinyl; and other natural and synthetic materials, such as nitrile, polyvinyl chloride, polyethylene, polyisoprene, neoprene, polychloriprene, etc. The gloves 12 may include other materials, such as antimicrobial coatings and/or coatings for protecting the skin that include aloe, chamomile, vitamin(s), or combinations thereof and other suitable ingredients that may provide skin care benefits, such as moisturizing and soothing dry, irritated skin. In addition to disposable gloves, it is contemplated that other types of gloves, i.e., non-medical or non-surgical gloves, could be used with the present concepts.

The packaging 10 comprises a barrier 22 that covers at least a portion of the opening 18 of the container 14. The barrier 22 may be a film, a piece of paper laminated with film or any type of flexible material that is suitable for providing or acting as a barrier. The barrier 22 inhibits or prevents microorganisms, pathogens, small airborne particles of dust and debris and other air contaminants from contacting and thus contaminating the gloves 12 prior to removal from the packaging 10. Thus, the barrier 22 helps to protect the gloves 12 from being exposed to undesirable materials or contaminants while the gloves 12 are in the packaging 10. Providing gloves 12 that are free from undesirable materials or contaminants reduces the risk that patients and healthcare workers will be exposed to such materials or contaminants and thereby reduces the opportunity to spread potentially harmful and infectious materials or contaminants. Furthermore, providing gloves 12 that are free from undesirable materials or contaminants also reduces or prevents cross-contamination that may occur between different patients.

In one embodiment, where the barrier 22 is a film, the film is a thin sheet of material, such as polypropylene, polystyrene, polyester, polyamide, polyvinylchloride, polyethylene (low density polyethylene, medium density polyethylene and/or high density polyethylene), polyvinylidene chloride, regenerated cellulose, cellulose acetate, and/or combinations thereof. The film material selected may be based on factors such as cost, shelf-life, barrier effectiveness, performance, etc. The film, including the paper-laminated film and any of the embodiments described herein, may have a thickness of less than about 0.254 mm (10 mils), particularly from about 0.025 mm to about 0.152 mm (1 mil to about 6 mils). The thickness may be selected based on a variety of factors such as barrier effectiveness, cost, material(s) used, performance characteristics such as transparency and flexibility, etc. The film may be clear or colored. The film may also be printed or plain, and may be flat, patterned or embossed. Also, the film may be laminated with one or more other materials, in addition to paper, such as foil, vinyl or other materials. The film, including the paper-laminated film and any of the embodiments described herein, helps to prevent exposure of the inside of the container 14 to microorganisms, airborne particles and other materials or contaminants.

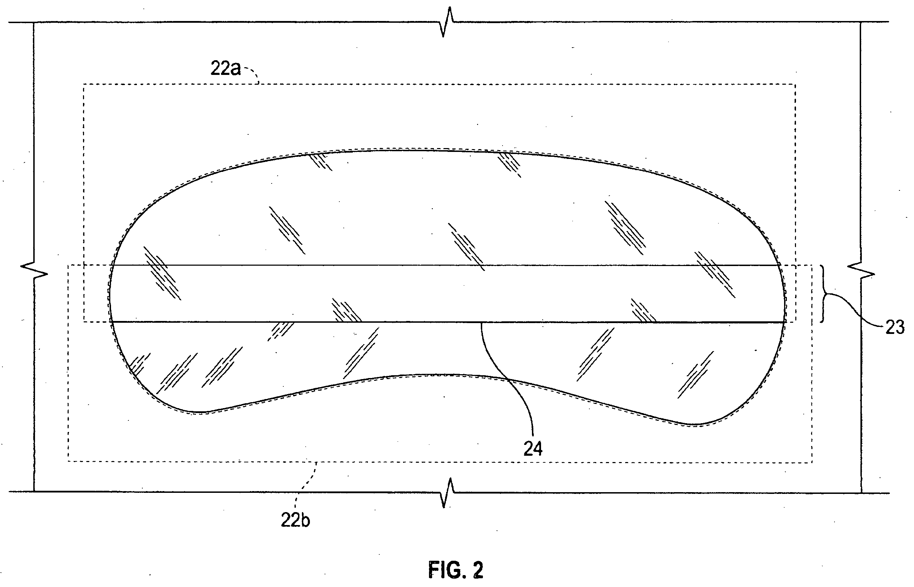

The barrier 22 may be a single sheet of film or may be multi-layered, as shown in FIGS. 1 and 2, as barriers 22a, 22b. The barriers 22, 22a, 22b may comprise the same type of or different materials. The barrier 22, 22a, 22b may also cover all or a portion of the top surface of the container 14, and may include an opening, aperture or slit in the middle of the barrier 22, 22a, 22b for removing the gloves 12. Having additional materials and/or additional layers may provide better protection than a single layer.

As shown in the embodiment of FIG. 2, the barriers 22a, 22b may include at least two overlapping films, including the paper-laminated film and any of the embodiments described herein, that are attached to the container 14. A first overlapping film or barrier 22a may cover a different portion of the opening 18 of the container 14 than a second overlapping film or barrier 22b. The barriers 22a, 22b may be positioned such that the barriers 22a, 22b are adjacent and overlap at an area corresponding approximately to the center of the opening 18, although it is contemplated that an overlapping portion 23 of the barriers 22a, 22b may be at other locations other than the center of the opening 18. Having the overlapping portions 23 at or near the center of the opening 18 may allow for larger areas for users to insert their hands to obtain one or more gloves 12, which may make removing the user's hand and glove easier. The overlapping portion 23 of the barriers 22a, 22b creates a slit 24 which allows a user to insert his or her hand through the slit 24 and remove a glove(s) 12 from the container 14. The barriers 22a, 22b may include a film, a piece of paper laminated with film or any type of flexible material that is suitable for providing or acting as a barrier.

Thus, one method for making the packaging 10 for gloves 12 includes providing a container 14 having a cavity 16 for holding the gloves 12 and an opening 18 for removing the gloves 12 from the container 14. Once a barrier, such as barrier 22, 22a, 22b, is provided which includes the antimicrobial material, a suitable method for attaching the barrier 22, 22a, 22b can be used to cover at least a portion of the opening 18 of the container 14. In some embodiments, the barrier 22, 22a, 22b includes overlapping films or barriers that cover the opening 18 of the container 14 and form the slit 24 for removal of the gloves 12 from the container 14.

In the embodiments shown in FIGS. 1 and 2, the barriers 22, 22a, 22b are attached to an inside surface of the top portion of the container 14. The barriers 22, 22a, 22b may be attached to the container 14 via adhesive material, glue, heat bonding, mechanical bonding, such as staples, and other suitable modes of attachment. The barriers 22, 22a, 22b may be attached along one or more peripheries, or other suitable area, of the top of the container 14. The barriers 22, 22a, 22b themselves may be made according to various methods, including extrusion, extrusion coating, co-extrusion or calendaring, or other suitable methods for making films. Extrusion, for example, is one of the most common and inexpensive methods for making a film.

The antimicrobial material may be added to the barrier 22, 22a, 22b according to different methods that include, but are not limited to, spraying, coating, mixing with a polymer before extrusion, or other methods suitable to result in an application or addition of the antimicrobial material to the barrier 22, 22a, 22b. The particular method chosen may depend on the type of manufacturing process being used to make the barrier 22, 22a, 22b, the end use of the product, cost and other relevant factors. In some embodiments, spraying may be the most cost effective method. In other embodiments, the antimicrobial material may be added to the barrier 22, 22a, 22b by mixing the antimicrobial material with the barrier material before extrusion. Mixing may be advantageous as it does not require additional steps in the manufacturing process. All of these methods provide for the antimicrobial material to be included on the surface of the barrier 22, 22a, 22b, distributed within the barrier 22, 22a, 22b, or both.

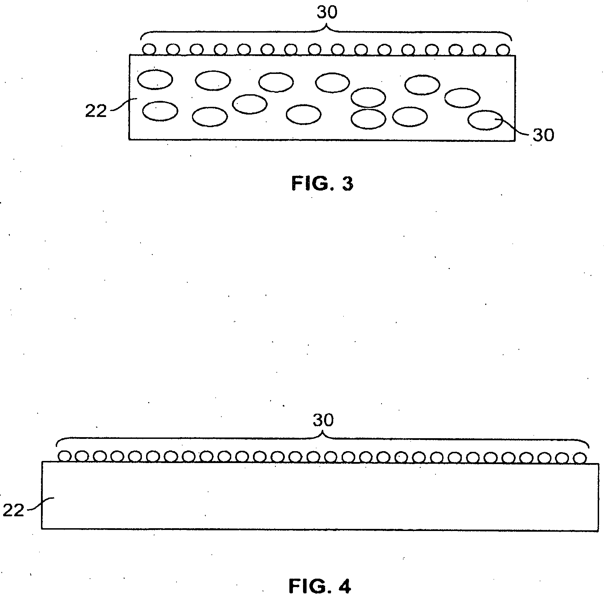

As shown in FIG. 3, the antimicrobial material 30 may be uniformly added to the surface of and incorporated within the barrier 22, 22a, 22b such that once a microorganism comes in contact with the barrier 22, 22a, 22b, it comes into contact with the antimicrobial material 30 and is killed. While it is possible to add the antimicrobial material 30 to only a portion of the barrier 22, 22a, 22b, such as coating the surface of the barrier 22, 22a, 22b with the antimicrobial material 30 as shown in FIG. 4, it is generally more economical and effective to treat the whole barrier 22, 22a, 22b (FIG. 3). In some embodiments, the antimicrobial material 30 distributed within the barrier 22, 22a, 2b may migrate to the surface of the barrier 22, 22a, 22b once the antimicrobial material 30 on the surface is consumed.

Thus, the antimicrobial material 30 that is added to the barrier 22, 22a, 22b destroys the microorganisms and pathogens that may come in contact with the barrier 22, 22a, 22b and thus reduces and/or eliminates the amount of microorganisms that may be deposited on the disposable gloves 12 housed within the container 14. The antimicrobial material 30 in combination with the barrier 22, 22a, 22b creates a contaminant-free zone on the packaging 10 that assists in reducing or eliminating patients and healthcare workers' exposure to potentially infectious and harmful microorganisms and contaminants. Additionally, the barrier 22, 22a, 22b prevents or reduces the occurrence of airborne particles and other materials or contaminants that may contaminate the gloves prior to removal from the packaging.

In accord with aspects of the disclosed concepts, the antimicrobial material 30 can be a selected blend of two or more additives which are shown to most effectively achieve a 4-log reduction (~99.9%) of priority microorganisms and contaminants. The test inoculum with which the effectiveness of the antimicrobial material 30 is shown may comprise, singly or in any combination, *Staphylococcus aureus* (or "S. aureus "; a common cause of staph infection, skin infections, respiratory disease, and food poisoning), methicillin resistant *Staphylococcus aureus* (or "MRSA"), *Klebsiella pneumonia* (or "K. pneumonia"; a form of bacterial pneumonia), *Escherichia coli* (commonly abbreviated "E. coli"; a well-known cause of serious food poisoning), *Pseudomonas aeruginosa* (or "P. aeruginosa"; a surface-borne bacteria with potentially fatal symptoms), or *Acinetobacter baumannii* (or "A. baumannii"; an antibiotic-resistant pathogenic bacteria that causes pneumonia and potentially fatal infections), for example. The test procedure with which the effectiveness of the antimicrobial material 30 is shown may include International Organization for Standardization (ISO) standard 22196:2007, "Plastics - Measurement of Antibacterial Activity on Plastics Surfaces," or ISO 22196:2011, "Measurement of antibacterial activity on plastics and other non-porous surfaces".

The "Value of Antimicrobial Activity" can be represented as:

$$R = [\log (B/C)]$$

where: R = value of antimicrobial activity; B = average of the number of viable cells of bacteria on the untreated test piece after 0 hours; and, C = average of the number of viable cells of bacteria on the antimicrobial test piece after 24 hours. R is commonly referred to as the "log reduction" of antimicrobial activity. It may be desirable, for some preferred embodiments, that the antimicrobial material 30 provide at least a 4-log reduction (i.e., less than approximately 0.1% survival rate) of all selected microorganisms and contaminants (e.g., those enumerated in the previous paragraph). By way of non-limiting example, some configurations may require the antimicrobial material 30 exhibit at least approximately a 4.9 log reduction of S. aureus, at least approximately a 4.9 log reduction of K. pneumonia, at least approximately a 6.0 log reduction of E. coli, and/or at least approximately a 5.2 log reduction of MRSA (e.g., as determined in accordance with ISO 22196:2007). For some configurations, the antimicrobial material 30 must exhibit at least approximately a 5.3 log reduction of MRSA, at least approximately a 5.1 log reduction of K. pneumonia, and/or at least approximately a 5.4 log reduction of E. coli (e.g., as determined in accordance with ISO 22196:2007). For some configurations, the antimicrobial material 30 must exhibit at least approximately a 5.6 log reduction of P. aeruginosa (e.g., as determined in accordance with ISO 22196:2007). Some configurations may require the antimicrobial material 30 exhibit at least approximately a 5.2 log reduction of A. baumannii (e.g., as determined in accordance with ISO 22196:2007).

According to the invention, the antimicrobial material 30 is a selected combination of zinc-based and silver-based materials which are proven, individually or in combination, to achieve the above-mentioned minimum 4-log reduction of microorganisms and contaminants. One preferred batch includes a mixture of a zinc-based antimicrobial compound, such as ULTRA-FRESH® KW-100 available from Thomson Research Associates, Inc., of Toronto, Ontario, Canada, at levels ranging from 0.075% to 0.1% of the total material weight, and a silver-based antimicrobial compound, such as ULTRA-FRESH® SA-18 available from Thomson Research Associates, Inc., at levels ranging from 0.1% to 0.5% of the total material weight. The silver-based antimicrobial compound may derive from the Silver Refractories chemical family, may have a specific gravity of approximately 2.7, may have a time-weighted average (TWA) of approximately 10 mg/m$^3$, may have a short term exposure limit (STEL) of 15 mg/m$^3$, and may comprise glass, oxide, and silver phosphate. In a similar regard, the zinc-based antimicrobial compound may derive from the Pyrithione chemical family, may have a specific gravity of approximately 1.8, may have a time-weighted average (TWA) of approximately 0.35 mg/m$^3$, and may comprise Bis(1-hydroxy-2(1H)-pyridinethionato-O,S)-(T-4) zinc and 2-Pyridinol-1-oxide. Optionally, the selected materials may exhibit bactericide, fungicide and/or algaecide characteristics.

As indicated above, the barrier 22 may be attached to the container 14 via an adhesive material. The adhesive may be a thermoplastic pressure sensitive adhesive (PSA), a hot-melt-adhesive, a thermoset adhesives, or any other adhesive with sufficient bonding strength for the intended application. It is desirable, for at least some applications, to pre-treat the barrier 22 prior to or during the attachment process in order to improve bonding strength and, thus, minimize any inadvertent detachment of the barrier 22 from the container 14. Applications where the barrier 22 is a polymeric film, for example, may require the contact side of the barrier, or selected portions thereof, be corona treated (also known as "air plasma" treated) to impart changes to the properties of the contact surface and thereby increase the surface tension of the film. Alternative methods by which the surface tension of the barrier 22 may be increased include etching, priming, flame treatment, and ozone treatment. For some applications, the film surface tension (or "dyne level") is increased to at least approximately 42 dynes or, in some embodiments at least approximately 48 dynes, to assist the barrier 22 in adhering to the glove box container 14. The surface tension may be measured in accordance with DIN ISO 8296 or American Society for Testing and Materials (ASTM) standard D2578-09. It may be desirable, for some embodiments, that the barrier 22 exhibits a minimum peel-force strength of 166-218 N/m or, for some embodiments, at least approximately 192 N/m. For some embodiments, the barrier 22 exhibits an average maximum pull-off force of at least approximately 184 N/m or, for some embodiments, at least approximately 230 N/m or, for some embodiments, at least approximately 275 N/m.

The peel force test for a thin antimicrobial film is carried out as follows: the thin film is sticked to paper, the cohesive paper is cut into 1.5mm strips in width, one end of the 1.5mm strip is fixed in an upper clamp, and the other end of the 1.5mm strip is fixed in a lower clamp. A standard textile tensile tester (ASTM D3822) is used to test the peel force of the thin film from the 1.5mm strip. In the experiment, it was found that the thin film cannot be peeled from the paper, finally, the paper is torn into pieces.

The container 14 of the glove packaging 10 may take on various shapes, sizes, and features within the scope of this disclosure. As shown in FIG. 1 of the drawings, for example, the container 14 is a rectangular polyhedron with opposing rectangular top and bottom walls that are interconnected by four rectangular sidewalls. The opening 18 of the glove packaging 10 is formed through the top wall of the container 14. The opening 18 may take on a variety of shapes, as indicated above; however, it may be desirable for some embodiments that the opening 18 have an elliptical shape with the major axis aligned parallel to the length of the container 14. The barrier 22 is, in at least some embodiments, a rectangular film that is adhered to the underside surface of the top wall of the container 14. For at least some embodiments, the top wall of the container is approximately 240 mm long x 124 mm high (9.4 in. x 4.9 in.), the film barrier is approximately 150 mm long x 112 mm high (5.9 in. x 4.4 in.), and/or the opening is approximately 120 mm long x 60 mm high (4.7 in. x 2.4 in.). Optionally, the opening can be approximately 122 mm long x 64 mm high (4.8 in. x 2.5 in.). Optionally, the film barrier is approximately 160 mm wide x 102mm long (6.3 in. x 4.0 in.) or approximately 191 mm wide x 102mm long (7.5 in. x 4.0 in.). In some embodiments, the adhesive is a thermoset Glue pattern: square parameter around the window perforation.

While the present invention has been described with reference to one or more particular embodiments, those skilled in the art will recognize that many changes may be made thereto without departing from the scope of the present invention. Each of these embodiments and obvious variations thereof is contemplated as falling within the scope of the invention, which is set forth in the following claims.

## EXAMPLES

The present invention is illustrated by the following examples.

### Preparation of a phosphate buffer solution

34.0 g of potassium dihydrogen phosphate are placed in a volumetric flask, and 500 ml of purified water or ion-exchanged water is added to dissolve the content sufficiently. The pH is adjusted to 6.8 to 7.2 (25 °C) with a sodium hydroxide solution. Further, purified water or ion-exchanged water is added to make 1000 ml. When the solution is used, the solution is dispensed into test tubes or Erlenmeyer flasks, cotton stoppers are put in, and it is sterilized with high-pressure steam. A phosphate buffer solution kept for one month or longer after preparation must not be used.

### Preparation of a phosphate buffered physiological saline

The phosphate buffer solution is diluted with physiological saline (0.85 % sodium chloride solution) into an 800-fold volume. When the solution is used, it is dispense into test tubes or Erlenmeyer flasks, cotton stoppers are put in, and it is sterilized with high-pressure steam. If the solution is not used immediately after preparation, it can be stored at 5 °C to 10 °C. A phosphate buffered physiological saline kept for one month or longer after preparation must not be used.

### High pressure steam sterilization

Water is placed in an autoclave, and the objects to be sterilized, which have been kept in a metal net basket, are put on a metal net shelf in the autoclave. The lid on the autoclave is tightened, the autoclave is heated, and kept at 121 °C temperature and 103 kPa pressures for 15 min to 20 min. The heating is stopped, it is allowed to cool down to lower than 100 °C, and an exhaust valve to draw off the steam is opened. The sterilized objects are taken out after opening the lid, and if necessary, the objects are further cooled on a clean bench or in a safety cabinet. In order to keep the autoclave clean from contamination by incubation or processing chemicals, it is washed with neutral detergent if needed, and rinsed sufficiently with water.

### Example 1: Test for antimicrobial activity

The test for antimicrobial activity is carried out as follows:

a). The untreated test piece and the antimicrobial test piece were sterilized according to the method mentioned in the high pressure steam sterilization.
b). 0.1 mL inoculum of *Pseudomonas aeruginosa* (ATCC #13388) is added in micro droplets on the surface of an untreated test piece and the antimicrobial test piece (the concentration of *Pseudomonas aeruginosa* are the same on both surfaces: $8.92 \times 10^5$ organisms/mL) at the same time.
c). The surface of the untreated test piece is washed by phosphate buffered physiological saline after the step b); the final solution is obtained, which is used to wash the untreated test piece. The number of bacteria in the final solution is count with direct microscopic observation as B1. The experiment from step a) to step c) is repeated twice to obtain B2 and B3. B is calculated as follows:

$$B= (B1+B2+B3)/3$$

d). the surface of the antimicrobial test piece from the step b) is kept for 24 hours, then the surface of the antimicrobial test piece is washed by phosphate buffered physiological saline, the final solution is obtained, which is used to wash the antimicrobial test piece. The number of bacteria in the final solution is count with direct microscopic observation as C1. The experiment from step a), step b) and step d) is repeated twice to obtain C2 and C3. C is calculated as follows:

6

$$C = (C1+C2+C3)/3$$

The "Value of Antimicrobial Activity" is calculated as follows:

$$R = [\log (B/C)]$$

Where: R = value of antimicrobial activity; B = average of the number of viable cells of bacteria on the untreated test piece after 0 hours; and, C = average of the number of viable cells of bacteria on the antimicrobial test piece after 24 hours. R is commonly referred to as the "log reduction" of antimicrobial activity.

Table 1 Value of Antimicrobial Activity

| Microorganism | *Pseudomonas aeruginosa* | | |
|---|---|---|---|
| Concentration of starting inoculum | $8.92 \times 10^5$ CFU/mL | | |
| | Bacteria number | | |
| Material | 0h | 24h | R = [log(B/C)] |
| untreated test piece | $5.75 \times 10^6$ | | |
| antimicrobial test piece | $5.75 \times 10^6$ | $<5.00 \times 10^1$ | >5.1 |

From the data in the table 1, it can be seen that the antimicrobial material 30 exhibits at least a 5.6 log reduction of *P. aeruginosa.*

**Example 2: Test for antimicrobial activity**

The test for antimicrobial activity is carried out as follows:

a). The untreated test piece and the antimicrobial test piece were sterilized according to the method mentioned in the high pressure steam sterilization.
b). 0.1 mL inoculum of *Escherichia coli* (ATCC #8739) is added in micro droplets on the surface of an untreated test piece and the antimicrobial test piece (the concentration of *Escherichia coli* are the same on both surfaces: $3.80 \times 10^5$ organisms/mL) at the same time.
c). The surface of the untreated test piece is washed by phosphate buffered physiological saline after the step b); the final solution is obtained, which is used to wash the untreated test piece. The number of bacteria in the final solution is count with direct microscopic observation as B1. The experiment from step a) to step c) is repeated twice to obtain B2 and B3. B is calculated as follows:

$$B = (B1+B2+B3)/3$$

d). the surface of the antimicrobial test piece from the step b) is kept for 24 hours, then the surface of the antimicrobial test piece is washed by phosphate buffered physiological saline, the final solution is obtained, which is used to wash the antimicrobial test piece. The number of bacteria in the final solution is count with direct microscopic observation as C1. The experiment from step a), step b) and step d) is repeated twice to obtain C2 and C3. C is calculated as follows:

$$C = (C1+C2+C3)/3$$

The "Value of Antimicrobial Activity" is calculated as follows:

$$R = [\log (B/C)]$$

EP 2 774 570 B1

Where: R = value of antimicrobial activity; B = average of the number of viable cells of bacteria on the untreated test piece after 0 hours; and, C = average of the number of viable cells of bacteria on the antimicrobial test piece after 24 hours. R is commonly referred to as the "log reduction" of antimicrobial activity.

Table 2 Value of Antimicrobial Activity

| Microorganism | *Escherichia coli* | | |
|---|---|---|---|
| **Concentration of starting inoculum** | 3.80x $10^5$ CFU/mL | | |
| | **Bacteria number** | | |
| **Material** | **0h** | **24h** | **R=[log(B/C)]** |
| untreated test piece | 1.91 x $10^6$ | | |
| antimicrobial test piece | 1.91 x $10^6$ | <5.00 x $10^1$ | >4.6 |

From the data in the table 2, it can be seen that the antimicrobial material 30 exhibits at least a 4.6 log reduction of *Escherichia coli.*

**Example 3: Test for antimicrobial activity**

The test for antimicrobial activity is carried out as follows:

a). The untreated test piece and the antimicrobial test piece were sterilized according to the method mentioned in the high pressure steam sterilization.
b). 0.1 mL inoculum of Methicillin Resistant *Staphylococcus aureus* (ATCC #33591) is added in micro droplets on the surface of an untreated test piece and the antimicrobial test piece (the concentration of Methicillin Resistant *Staphylococcus aureus* are the same on both surfaces: $3.20 \times 10^5$ organisms/mL) at the same time.
c). The surface of the untreated test piece is washed by phosphate buffered physiological saline after the step b); the final solution is obtained, which is used to wash the untreated test piece. The number of bacteria in the final solution is count with direct microscopic observation as B1. The experiment from step a) to step c) is repeated twice to obtain B2 and B3. B is calculated as follows:

$$B= (B1+B2+B3)/3$$

d). the surface of the antimicrobial test piece from the step b) is kept for 24 hours, then the surface of the antimicrobial test piece is washed by phosphate buffered physiological saline, the final solution is obtained, which is used to wash the antimicrobial test piece. The number of bacteria in the final solution is count with direct microscopic observation as C1. The experiment from step a), step b) and step d) is repeated twice to obtain C2 and C3. C is calculated as follows:

$$C= (C1+C2+C3)/3$$

The "Value of Antimicrobial Activity" is calculated as follows:

$$R = [\log (B/C)]$$

Where: R = value of antimicrobial activity; B = average of the number of viable cells of bacteria on the untreated test piece after 0 hours; and, C = average of the number of viable cells of bacteria on the antimicrobial test piece after 24 hours. R is commonly referred to as the "log reduction" of antimicrobial activity.

Table 3 Value of Antimicrobial Activity

| Microorganism | Methicillin Resistant *staphylococcus aureus* |
|---|---|
| **Concentration of starting inoculum** | 3.20x $10^5$ CFU/mL |

(continued)

| Material | Bacteria number | | R = [log(B/C)] |
|---|---|---|---|
| | 0h | 24h | |
| untreated test piece | $1.10 \times 10^6$ | | |
| antimicrobial test piece | $1.10 \times 10^6$ | $<5.00 \times 10^1$ | >4.3 |

From the data in the table 3, it can be seen that the antimicrobial material 30 exhibits at least a 4.3 log reduction of Methicillin Resistant *Staphylococcus aureus.*

## Claims

1.   A packaging (10) for holding and dispensing gloves (12), the packaging (10) comprising:

a container (14) with a cavity (16) configured to hold the gloves (12), the container (14) having an opening (18) through which the gloves (12) are removable from the container (14); and
a barrier (22) attached to the container (14) and covering at least a portion of the opening (18) of the container (14), the barrier (22) including an antimicrobial material, wherein the antimicrobial material includes a combination of zinc-based and silver-based materials
**characterized in that** the combination of zinc-based and silver-based materials collectively exhibit a minimum 4-log reduction of one or more predetermined pathogens, contaminants, or both; and
**in that** the antimicrobial material includes 0.075% to 0.1 % of total weight of the zinc-based material and 0.1 % to 0.5% of total weight of the silver-based material.

2.   The packaging (10) of claim 1, wherein the zinc-based material is an antimicrobial compound with a specific gravity of 1.8 and a time-weighted average (TWA) of 0.35 mg/m$^3$, and the silver-based material is an antimicrobial compound with a specific gravity of 2.7 and a time-weighted average (TWA) of 10 mg/m$^3$.

3.   The packaging (10) of claim 1 or 2, wherein the silver-based material comprises glass, oxide, and silver phosphate, and wherein the zinc-based material comprises Bis(1-hydroxy-2(1H)-pyridinethionato-O,S)-(T-4) zinc and 2-Pyridinol-1-oxide.

4.   The packaging (10) of any one of claims 1 to 3, wherein the antimicrobial material exhibits a minimum 4-log reduction of *Staphylococcus aureus* (S. aureus), methicillin resistant *Staphylococcus aureus* (MRSA), *Klebsiella pneumonia* (K. pneumonia), *Escherichia coli* (E. coli), *Pseudomonas aeruginosa,* or *Acinetobacter baumannii,* or any combinations thereof.

5.   The packaging (10) of any one of claims 1 to 4, wherein the antimicrobial material exhibits at least a 4.9 log reduction of S. aureus, at least a 4.9 log reduction of K. pneumonia, at least a 6.0 log reduction of E. coli, and at least a 5.2 log reduction of MRSA.

6.   The packaging (10) of any one of claims 1 to 5, wherein the antimicrobial material further exhibits bactericide, fungicide, or algaecide characteristics, or any combinations thereof.

7.   The packaging (10) of any one of claims 1 to 6, wherein at least a portion of the barrier (22) is corona treated to thereby increase the surface tension of the barrier.

8.   The packaging (10) of any one of claims 1 to 7, wherein at least a portion of the barrier (22) is treated to thereby increase the surface tension of the barrier (22) to at least 42 dynes.

9.   The packaging (10) of any one of claims 1 to 8, wherein the barrier (22) exhibits a minimum peel-force strength of at least 166 N/m.

10.   The packaging (10) of any one of claims 1 to 9, wherein the barrier (22) exhibits an average maximum pull-off force of at least 184 N/m.

**11.** A method for making a packaging (10) for gloves (12), the method comprising:

receiving a container (14) having a cavity (16) configured to hold the gloves (12), the container (14) having an opening (18) through which the gloves (12) are removable from the container (14);

receiving a barrier (22) having an antimicrobial material, wherein the antimicrobial material includes a predetermined combination of zinc-based and silver-based materials and

attaching the barrier (22) to the container (14) such that the barrier (22) covers at least a portion of the opening (18) of the container (14); **characterized in that** the combination of zinc-based and silver-based materials collectively exhibit a minimum 4-log reduction of one or more predetermined pathogens, contaminants, or both; and

**in that** the antimicrobial material includes 0.075% to 0.1% of total weight of the zinc-based material and 0.1% to 0.5% of total weight of the silver-based material.

**12.** The method of claim 11, wherein the zinc-based material is an antimicrobial compound with a specific gravity of 1.8 and a time-weighted average (TWA) of 0.35 mg/m$^3$, and the silver-based material is an antimicrobial compound with a specific gravity of 2.7 and a time-weighted average (TWA) of 10 mg/m$^3$.

**13.** The method of claim 11 or 12, wherein the antimicrobial material exhibits a minimum 4-log reduction of *Staphylococcus aureus* (S. aureus), methicillin resistant *Staphylococcus aureus* (MRSA), *Klebsiella pneumonia* (K. pneumonia), *Escherichia coli* (E. coli), *Pseudomonas aeruginosa,* and *Acinetobacter baumannii.*

**14.** The method of any one of claims 11 to 13, further comprising treating at least a portion of the barrier (22) to thereby increase the surface tension of the barrier (22) to at least 42 dynes.

**15.** The method of any one of claims 11 to 14, wherein the attaching includes adhering the barrier (22) to the container (14) such that the barrier exhibits a minimum peel-force strength of at least 166 N/m.

**Patentansprüche**

**1.** Verpackung (10) zum Halten und Ausgeben von Handschuhen (12), wobei die Verpackung (10) umfasst:

ein Behältnis (14) mit einem Hohlraum (16), gestaltet um die Handschuhe (12) zu halten, wobei das Behältnis (14) eine Öffnung (18) besitzt, durch welche die Handschuhe (12) aus dem Behältnis (14) entnehmbar sind; und

eine Barriere (22), befestigt an dem Behältnis (14) und mindestens einen Teil der Öffnung (18) des Behältnisses (14) bedeckend, wobei die Barriere (22) ein antimikrobielles Material beinhaltet, wobei das antimikrobielle Material eine Kombination aus Zink-basierten und Silber-basierten Materialien beinhaltet, **dadurch gekennzeichnet, dass** die Kombination aus Zink-basierten und Silber-basierten Materialien zusammen eine mindestens 4 log Reduktion von einem oder mehreren vorbestimmten Erregern, Kontaminanten oder beiden aufweist; und dass das antimikrobielle Material 0,075% bis 0,1% des Gesamtgewichts an Zink-basierten Material und 0,1% bis 0,5% des Gesamtgewichts an Silber-basierten Material beinhaltet.

**2.** Verpackung (10) nach Anspruch 1, wobei das Zink-basierte Material eine antimikrobielle Verbindung mit einem spezifischen Gewicht von 1,8 und einem zeitgewichteten Durchschnitt (TWA) von 0,35 mg/m$^3$ ist, und das Silber-basierte Material eine antimikrobielle Verbindung mit einem spezifischen Gewicht von 2,7 und einem zeitgewichteten Durchschnitt (TWA) von 10 mg/m$^3$ ist.

**3.** Verpackung (10) nach Anspruch 1 oder 2, wobei das Silber-basierte Material Glas, Oxid und Silberphosphat umfasst, und wobei das Zink-basierte Material Bis(1-hydroxy-2(1H)-pyridinethionato-O,S)-(T-4)zink und 2-Pyridinol-1-oxid umfasst.

**4.** Verpackung (10) nach einem der Ansprüche 1 bis 3, wobei das antimikrobielle Material mindestens eine 4 log Reduktion von *Staphylococcus aureus* (S. aureus), *Methicillin-resistenter Staphylococcus aureus* (MRSA), *Klebsiella pneumonia* (K. pneumonia), *Escherichia coli* (E. coli), *Pseudomonas aeruginosa* oder *Acinetobacter baumannii,* oder beliebige Kombinationen davon aufweist.

**5.** Verpackung (10) nach einem der Ansprüche 1 bis 4, wobei das antimikrobielle Material mindestens eine 4,9 log Reduktion von S. aureus, mindestens eine 4,9 log Reduktion von K. pneumonia, mindestens eine 6,0 log Reduktion von E. coli und mindestens eine 5,2 log Reduktion von MRSA aufweist.

6. Verpackung (10) nach einem der Ansprüche 1 bis 5, wobei das antimikrobielle Material ferner bakterizide, fungizide oder algizide Merkmale oder beliebige Kombinationen davon aufweist.

7. Verpackung (10) nach einem der Ansprüche 1 bis 6, wobei mindestens ein Teil der Barriere (22) koronabehandelt ist, um dadurch die Oberflächenspannung der Barriere zu erhöhen.

8. Verpackung (10) nach einem der Ansprüche 1 bis 7, wobei mindestens ein Teil der Barriere (22) behandelt ist, um dadurch die Oberflächenspannung der Barriere (22) auf mindestens 42 dyn zu erhöhen.

9. Verpackung (10) nach einem der Ansprüche 1 bis 8, wobei die Barriere (22) eine Mindestschälkraftfestigkeit von mindestens 166 N/m aufweist.

10. Die Verpackung (10) nach einem der Ansprüche 1 bis 9, wobei die Barriere (22) eine durchschnittliche maximale Abzugskraft von mindestens 184 N/m aufweist.

11. Verfahren zur Herstellung einer Verpackung (10) für Handschuhe (12), wobei das Verfahren umfasst:

Nehmen eines Behältnisses (14) mit einem Hohlraum (16), gestaltet um die Handschuhe (12) zu halten, wobei das Behältnis (14) eine Öffnung (18) besitzt, durch welche die Handschuhe (12) aus dem Behältnis (14) entnehmbar sind
Nehmen einer Barriere (22) mit einem antimikrobiellen Material, wobei das antimikrobielle Material eine vorbestimmte Kombination aus Zink-basierten und Silber-basierten Materialien beinhaltet; und
Befestigen der Barriere (22) an dem Behältnis (14), so dass die Barriere (22) mindestens einen Teil der Öffnung (18) des Behältnisses (14) abdeckt; **dadurch gekennzeichnet, dass** die Kombination aus Zink-basierten und Silber-basierten Materialien zusammen eine mindestens 4 log Reduktion von einem oder mehreren vorbestimmten Erregern, Kontaminanten oder beiden aufweist; und
dass das antimikrobielle Material 0,075% bis 0,1% des Gesamtgewichts an Zink-basierten Material und 0,1% bis 0,5% des Gesamtgewichts an Silber-basierten Material beinhaltet.

12. Verfahren nach Anspruch 11, wobei das Zink-basierte Material eine antimikrobielle Verbindung mit einem spezifischen Gewicht von 1,8 und einem zeitgewichteten Durchschnitt (TWA) von 0,35 mg/m$^3$ ist, und das Silber-basierte Material eine antimikrobielle Verbindung mit einem spezifisches Gewicht von 2,7 und einem zeitgewichteten Durchschnitt (TWA) von 10 mg/m$^3$ ist.

13. Verfahren nach Anspruch 11 oder 12, wobei das antimikrobielle Material mindestens eine 4 log Reduktion von *Staphylococcus aureus* (S. aureus), *Methicillin-resistenter Staphylococcus aureus* (MRSA), *Klebsiella pneumonia* (K. pneumonia), *Escherichia coli* (E. coli), *Pseudomonas aeruginosa* oder *Acinetobacter baumannii,* oder beliebige Kombinationen davon aufweist.

14. Verfahren nach einem der Ansprüche 11 bis 13, ferner umfassend das Behandeln mindestens eines Teils der Barriere (22), um dadurch die Oberflächenspannung der Barriere (22) auf mindestens 42 dyn zu erhöhen.

15. Verfahren nach einem der Ansprüche 11 bis 14, wobei das Anhängen der Barriere (22) an das Behältnis (14) umfasst, derart dass die Barriere eine Mindestschälkraftfestigkeit von mindestens 166 N/m aufweist.

**Revendications**

1. Emballage (10) de maintien et de distribution de gants (12), l'emballage (10) comprenant : un récipient (14) ayant une cavité (16) configurée pour contenir les gants (12), le récipient (14) présentant une ouverture (18) à travers laquelle les gants (12) peuvent être retirés du récipient (14) ; et
une barrière (22) fixée au récipient (14) et recouvrant au moins une partie de l'ouverture (18) du récipient (14), la barrière (22) comprenant un matériau antimicrobien, où le matériau antimicrobien comprend une combinaison de matériaux à base de zinc et à base d'argent **caractérisée en ce que** la combinaison des matériaux à base de zinc et à base d'argent fait preuve collectivement d'une réduction minimale de 4 log d'un ou plusieurs pathogènes, contaminants, prédéterminés ou les deux ; et

**en ce que** le matériau antimicrobien comprend 0,075 % à 0,1 % du poids total du matériau à base de zinc et 0,1 % à 0,5 % du poids total du matériau à base d'argent.

2. Emballage (10) selon la revendication 1, où le matériau à base de zinc est un composé antimicrobien présentant une densité relative de 1,8 et une moyenne pondérée dans le temps (TWA) de 0,35 mg/m$^3$, et le matériau à base d'argent est un composé antimicrobien présentant une densité relative de 2,7 et une moyenne pondérée dans le temps (TWA) de 10 mg/m$^3$.

3. Emballage (10) selon la revendication 1 ou 2, où le matériau à base d'argent comprend du verre, un oxyde, et du phosphate d'argent, et où le matériau à base de zinc comprend du bis(1-hydroxy-2(1H)-pyridinethionato-O,S) - (T-4) zinc et du 1-oxyde de 2-pyridinol.

4. Emballage (10) selon l'une quelconque des revendications 1 à 3, où le matériau antimicrobien fait preuve d'une réduction minimale de 4 log de *Staphylococcus aureus* (S. aureus), de *Staphylococcus aureus* résistant à la méthicilline (MRSA), de *Klebsiella pneumonia* (K. pneumonia), d'*Escherichia coli* (E. coli), de *Pseudomonas aeruginosa,* ou d'*Acinetobacter baumannii,* ou n'importe quelles combinaisons de ceux-ci.

5. Emballage (10) selon l'une quelconque des revendications 1 à 4, où le matériau antimicrobien fait preuve d'une réduction d'au moins 4,9 log de *S. aureus,* d'au moins une réduction de 4,9 log de *K. pneumonia,* d'au moins une réduction de 6,0 log d'E. *coli,* et d'au moins une réduction de 5,2 log de MRSA.

6. Emballage (10) selon l'une quelconque des revendications 1 à 5, où le matériau antimicrobien fait preuve en outre de caractéristiques bactéricides, fongicides, ou algicides, ou de n'importe quelles combinaisons de celles-ci.

7. Emballage (10) selon l'une quelconque des revendications 1 à 6, où au moins une partie de la barrière (22) est traitée de manière couronne et augmente de là la tension superficielle de la barrière.

8. Emballage (10) selon l'une quelconque des revendications 1 à 7, où au moins une partie de la barrière (22) est traitée pour accroître ainsi la tension superficielle de la barrière (22) jusqu'à au moins 42 dynes.

9. Emballage (10) selon l'une quelconque des revendications 1 à 8, où la barrière (22) fait preuve d'une résistance minimale au décollement d'au moins 166 N/m.

10. Emballage (10) selon l'une quelconque des revendications 1 à 9, où la barrière (22) fait preuve d'une force maximale moyenne d'arrachement d'au moins 184 N/m.

11. Procédé de fabrication d'un emballage (10) pour des gants (12), le procédé comprenant :

la réception d'un récipient (14) présentant une cavité (16) configurée pour contenir les gants (12), le récipient (14) présentant une ouverture (18) à travers laquelle les gants (12) peuvent être retirés du récipient (14) ;
la réception d'une barrière (22) possédant un matériau antimicrobien, où le matériau antimicrobien comprend une combinaison de matériaux à base de zinc et à base d'argent **caractérisée en ce que** la combinaison des matériaux à base de zinc et à base d'argent fait preuve collectivement d'une réduction minimale de 4 log d'un ou plusieurs pathogènes, contaminants, prédéterminés ou les deux ; et
et la fixation de la barrière (22) au récipient (14) de sorte que la barrière (22) recouvre au moins une partie de l'ouverture (18) du récipient (14) ;
**en ce que** le matériau antimicrobien comprend 0,075 % à 0,1 % du poids total du matériau à base de zinc et 0,1 % à 0,5 % du poids total du matériau à base d'argent.

12. Procédé selon la revendication 11, où le matériau à base de zinc est un composé antimicrobien présentant une densité relative de 1,8 et une moyenne pondérée dans le temps (TWA) de 0,35 mg/m$^3$, et le matériau à base d'argent est un composé antimicrobien présentant une densité relative de 2,7 et une moyenne pondérée dans le temps (TWA) de 10 mg/m$^3$.

13. Procédé selon la revendication 11 ou 12, où le matériau antimicrobien fait preuve d'une réduction minimale de 4 log de *Staphylococcus aureus* (S. aureus), de *Staphylococcus aureus* résistant à la méthicilline (MRSA), de *Klebsiella pneumonia* (K. pneumonia), d'*Escherichia coli* (E. coli), de *Pseudomonas aeruginosa,* et d'*Acinetobacter baumannii.*

**14.** Procédé selon l'une quelconque des revendications 11 à 13, comprenant en outre le traitement d'au moins une partie de la barrière (22) pour accroître de là la tension superficielle de la barrière (22) jusqu'à au moins 42 dynes.

**15.** Procédé selon l'une quelconque des revendications 11 à 14, où la fixation comprend l'adhésion de la barrière (22) au récipient (14) de sorte que la barrière fait preuve d'une résistance minimale au décollement d'au moins 166 N/m.

FIG. 1

FIG. 2

**FIG. 3**

**FIG. 4**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009129182 A1